# EUROPEAN PATENT APPLICATION

(11) **EP 3 892 739 A1**
(43) Date of publication of application: **13.10.2021**
(21) Application number: 20315145.1
(22) Date of filing: 09.04.2020
(51) Int. Cl.: C12Q 1/6886

(54) **TYPE III INTERFERON FOR USE AS A BIOMARKER TO PREDICT RESPONSE TO A CANCER TREATMENT**

(71) Applicant: Centre Léon Bérard, 69008 Lyon (FR); INSTITUT NATIONAL DE LA SANTE ET DE LA RECHERCHE MEDICALE - INSERM, 75013 Paris (FR); Centre National de la Recherche Scientifique, 75016 Paris (FR); Université Claude Bernard Lyon 1, 69100 Villeurbanne (FR)
(72) Inventor: Caux, Christophe, 01360 Bressolles (FR); Valladeau-Guilemond, Jenny, 69970 Marennes (FR); Hubert, Margaux, 69008 Lyon (FR)
(74) Representative: Lavoix

(57) **Abstract**

The present invention relates to type III interferon, alone or in conjunction with Dendritic Cells subtypes markers, for use as a biomarker to predict response to a cancer treatment chosen from immunotherapies, targeted therapies, chemotherapies, and/or radiotherapies. It further relates to a method for predicting cancer treatment responsiveness, comprising detecting the expression of type III interferon in a sample from a subject, and to a type III interferon inducer for use in combination with an immunotherapy, targeted therapy, chemotherapy, and/or radiotherapy.

## Description

The present invention relates to type III interferon, alone or in conjunction with Dendritic Cells subtypes markers, for use as a biomarker to predict response to a cancer treatment chosen from immunotherapies, targeted therapies, chemotherapies, and/or radiotherapies. It further relates to a method for predicting cancer treatment responsiveness, comprising detecting the expression of type III interferon in a sample from a subject, and to a type III interferon inducer for use in combination with an immunotherapy, targeted therapy, chemotherapy, and/or radiotherapy.

Cancer is one of the major health problems in developed countries today. Cancer is an unregulated proliferation of cells due to loss of normal controls, resulting in unregulated growth, lack of differentiation, local tissue invasion, and, often, metastasis. Cancer can develop in any tissue or organ at any age.

Novel antitumor immunotherapies such as monoclonal antibodies targeting immune checkpoints (ICPs) has led to promising results in several cancers. However, despite the favorable outcome of responding patients, the response rate remains relatively low.

A major ongoing challenge is therefore the identification of biomarkers of response and of new immunotherapy targets/strategies for none-responding patients.

Type III interferon share the same signaling pathway as type I interferon, leading to the transcription of multiple interferon-stimulated genes (ISGs). Similar to type I interferon, type III interferon was shown to play a crucial role in autoimmune diseases and viral infections. Its antitumor activity was also reported in few mouse models. In human, its anti-proliferative and pro-apoptotic activities have so far only been demonstrated *in vitro.*

Owing to its specificity of action on a narrow range of cell types such as epithelial cells and some immune populations, targeting type III interferon may pave the way for novel interferon therapies with potentially limited toxicity compared to type I interferon treatments.

The inventors of the present invention have investigated the potential production of type III interferon by tumor-associated cDC1 and highlighted protective properties through antitumor immunity.

More particularly, the inventors of the present invention have demonstrated that type III interferon are associated to a favorable outcome in cancer treatments.

The present invention thus relates to a type III interferon for use as a biomarker to predict response to a cancer treatment chosen from immunotherapies, targeted therapies, chemotherapies, and/or radiotherapies.

The present invention also relates to method for predicting cancer treatment responsiveness, wherein said cancer treatment is chosen from immunotherapies, targeted therapies, chemotherapies, and/or radiotherapies and wherein said method comprises:
(a) detecting the expression of type III interferon in a sample from a subject; and
(b) deducing therefrom if the subject will reply positively to a cancer treatment by immunotherapy, targeted therapy, chemotherapy, and/or radiotherapy.

It further relates to a type III interferon inducer for use in combination with an immunotherapy, targeted therapy, chemotherapy, and/or radiotherapy for the treatment of cancer.

A further object of the invention is also a method of treatment of cancer, said method comprising the administration of a type III interferon inducer in combination with an immunotherapy, targeted therapy, chemotherapy, and/or radiotherapy.

In particular, said method comprises a first step of detection of the expression of type III interferon in a sample from a subject, and a second step of treatment with an immunotherapy, targeted therapy, chemotherapy, and/or radiotherapy, especially in the case where the expression of type III interferon is detected in said sample.

Definitions that follows will apply equally to the type III interferon for use according to the invention, the methods according to the invention and the type III interferon inducer for use according to the invention.

As used herein, a "biomarker" is a set of gene expression (e.g protein or mRNA) that it correlated to a positive outcome in the treatment of cancer with immunotherapies, targeted therapies, chemotherapies, and/or radiotherapies. Such positive biomarker is a type III interferon. More particularly, an expression of type III interferon is indicative of a positive outcome in the treatment of cancer by the aforesaid therapies. As will be detailed below, such biomarker can be used in combination with at least one additional biomarker.

By "type III interferon" is meant one type III interferon of the group of three IFN-λ (lambda) molecules called IFN-λ1, IFN-λ2, IFN-λ3 (also known as IL29, IL28A and IL28B respectively). These interferons signal through a receptor complex consisting of IL10RB (also called IL10R2 or CRF2-4) and IFNLR1 (formerly called IL28RA, CRF2-12). The interferon lambda genes lie in the 19q13.13 chromosomal region.

As used herein and unless otherwise defined, "cancer" refers to the growth, division or proliferation of abnormal cells in the body. Such cancers include, but are not limited to solid cancers, in particular those chosen from breast cancer, ovarian cancer, melanoma, lung cancer, colorectal carcinoma, Head and Neck squamous cell carcinoma, kidney carcinoma, liver hepatocellular carcinoma, thyroid cancer.

The terms "treat", "treating", "treated" or "treatment", as used herein, refer to therapeutic treatment wherein the object is to eliminate or lessen symptoms. Beneficial or desired clinical results include, but are not limited to, elimination of symptoms, alleviation of symptoms, diminishment of extent of condition, stabilized (i.e., not worsening) state of condition, delay or slowing of progression of the condition, to the prevention of the onset, recurrence or spread of a disease or disorder, or of one or more symptoms thereof. In certain embodiments, the terms refer to the treatment with or administration of a compound provided herein prior to the onset of symptoms. The terms encompass the inhibition or reduction of a symptom of the particular disease. Subjects with familial history of a disease in particular are candidates for treatment regimens in certain embodiments. Also, subjects in whom a genetic disposition for the particular disease has been shown are candidates for treatment regimens in certain embodiments. In addition, subjects who have a history of recurring symptoms are also potential candidates for the treatment. In this regard, the term "treatment" may be interchangeably used with the term "prophylactic treatment."

As used herein, "immunotherapy" is a type of therapy that uses substances to stimulate or suppress the immune system to help the body to fight cancer, in particular solid cancers, and more particularly those previously mentioned. Some types of immunotherapy only target certain cells of the immune system. Others affect the immune system in a general way. Types of immunotherapy encompassed by the present invention include anti-PD1, anti-CTLA-4, other antagonist or agonist antibody directed against T cells or other immune cells "immune check points", immunostimulatory agents such as PRR agonist (TLR ligands, RLR ligands, STING agonists), viruses in particular oncolytic viruses, and/or vaccine strategies.

As used therein, "targeted therapy" is a type of treatment that uses drugs or other substances to identify and attack specific types of cancer cells with less harm to normal cells. Some targeted therapies block the action of certain enzymes, proteins, or other molecules involved in the growth and spread of cancer cells. Other types of targeted therapies help the immune system kill cancer cells or deliver toxic substances directly to cancer cells and kill them. Targeted therapy may have fewer side effects than other types of cancer treatment. Most targeted therapies are either small molecule drugs or monoclonal antibodies.

In the context of the invention, targeted therapy will attack specific types of cancer cells, in particular those of solid cancers and more particularly cancer cells of those cancers previously mentioned.

Examples of targeted therapy encompassed by the present invention are inhibitors of receptor tyrosine kinases (HER2, cKIT, VEGFR, TGFbR, ...), of cellular kinases (BRAF, MAP kinases,..) or other enzymes (PRMT, IDO, CD73,...), or antibodies against cell surface receptors (anti-HER2, anti-EGFR, anti-CD20, ...) or secreted molecules (anti-VEGF, anti-Netrin,...).

By "chemotherapy" is meant the medical use of a cytotoxic agent that is known to be of use in chemotherapy for cancer, in the context of the invention chemotherapy known to treat in particular solid cancers, more particularly those previously mentioned.

By "radiotherapy", "radiation therapy", or "radiation oncology", often abbreviated RT, RTx, or XRT, is meant the medical use of ionizing radiation, generally as part of cancer treatment to control or kill malignant cells.

In the context of the invention, the biomarker is used to predict response to a cancer treatment chosen from immunotherapies, targeted therapies, chemotherapies, and/or radiotherapies. In particular, it allows to predict if the subject will reply positively to said treatment or not.

As used herein "reply positively" or "positive outcome" means that the treatment with an immunotherapy, targeted therapy, chemotherapy, and/or radiotherapy will most probably be effective i.e. will eliminate symptoms, alleviate symptoms, diminish extent of condition, stabilized (i.e., not worsening) state of condition, delay or slow progression of the condition, prevention the onset, recurrence or spread of a the disease, or of one or more symptoms thereof.

In one embodiment, said Type III interferon is expressed by cDC1.

"cDC1" means conventional type 1 dendritic cells and these terms will be used herein interchangeably.

In the context of the invention, type III interferon can be used in combination with at least one additional biomarker, in particular Dendritic Cells subtypes markers. In particular, said additional biomarker is chosen from: tumor mutational burden (TMB), neoAg load, CD8 T cells infiltration, PDL1 expression, cDC1 mRNA signature, CLEC9A and XCR1 proteins or mRNA. More particularly, said additional biomarker is chosen from cDC1 mRNA signature or CLEC9A and/or XCR1 proteins or mRNA.

"Tumor mutational burden (TMB)" is known by the skilled person. It consists in a measurement of mutations carried by tumor cells. This biomarker can be measured according to the methods detailed for example in Chan TA et al. ("Development of tumor mutation burden as an immunotherapy biomarker: utility for the oncology clinic", Ann Oncol. 2019 Jan 1;30(1):44-56. doi: 10.1093/annonc/mdy495. Review., PMID:30395155), Büttner R et al. ("Implementing TMB measurement in clinical practice: considerations on assay requirements", ESMO Open. 2019 Jan 24;4(1):e000442. doi: 10.1136/esmoopen-2018-000442. eCollection 2019, PMID:30792906) and Fancello L et al. ("Tumor mutational burden quantification from targeted gene panels: major advancements and challenges", J Immunother Cancer. 2019 Jul 15;7(1):183. doi: 10.1186/s40425-019-0647-4. Review, PMID:31307554).

« NeoAg load" or « neoantigens load » corresponds to tumor specific mutations in peptides, from protein expressed by the tumor cells, and that can be presented in the Major Histocompatibility Complex (CMH) of the patient. This biomarker can be measured according to the methods detailed for example in Schumacher TN et al ("Neoantigens in cancer immunotherapy", Science. 2015 Apr 3;348(6230):69-74. doi: 10.1126/science.aaa4971. Review., PMID:25838375), Roudko V et al. ("Computational Prediction and Validation of Tumor-Associated Neoantigens", Front Immunol. 2020 Jan 24;11:27. doi: 10.3389/fimmu.2020.00027. eCollection 2020. Review, PMID:32117226) and Alcazer V et al., "Neoepitopes-based vaccines: challenges and perspectives.", Eur J Cancer. 2019 Feb;108:55-60. doi: 10.1016/j.ejca.2018.12.011. Epub 2019 Jan 12. PMID:30648630).

« CD8 T cells infiltration » corresponds to the density of CD8 T cells infiltrating the tumors usually define by immunostaining on FFPE tumor sections . This biomarker can be measured according to the methods detailed for example in Pages F et al. ("International validation of the consensus Immunoscore for the classification of colon cancer: a prognostic and accuracy study.", Lancet. 2018 May 26;391(10135):2128-2139. doi: 10.1016/S0140-6736(18)30789-X. Epub 2018 May 10, PMID:29754777), Salgado R et al. ("The evaluation of tumor-infiltrating lymphocytes (TILs) in breast cancer: recommendations by an International TILs Working Group 2014", Ann Oncol. 2015 Feb;26(2):259-71. doi: 10.1093/annonc/mdu450. Epub 2014 Sep 11, PMID:25214542), Tumeh PC et al. ("PD-1 blockade induces responses by inhibiting adaptive immune resistance", Nature. 2014 Nov 27;515(7528):568-71. doi: 10.1038/nature13954, PMID:25428505) and Bonaventura P et al. ("Cold Tumors: A Therapeutic Challenge for Immunotherapy", Front Immunol. 2019 Feb 8;10:168. doi: 10.3389/fimmu.2019.00168. eCollection 2019. Review, PMID:30800125).

« cDC1 mRNA signature» is meant to determine the cDC1 mRNA signature which corresponds to the mRNA profile of conventional type 1 dendritic cells. It can be measured by any method known by the man skilled the art such as mRNA sequencing and appropriate bioinformatics deconvolution tools .

"Proteins CLEC9A and XCR1" are known in the art, their expression is highly selective to cDC1. CLEC9A corresponds to the C-type lectin domain family 9 member A and is a protein that in humans is encoded by the CLEC9A gene. The sub-family of chemokine receptors contains only one member: XCR1, the receptor for XCL1 and XCL2 (or lymphotactin-1 and -2). XCR1 is also known as GPR5. The expression of these proteins or their respective mRNA can be measured by any method know by the man skilled in the art such as in situ hybridization using mRNA probes, immunofluorescence in situ multiparametric flow-cytometry using antibodies, RT-PCR, RNAseq, ...

All these additional biomarkers are predictive of a positive outcome for the treatment of cancer with an immunotherapy, targeted therapy, chemotherapy, and/or radiotherapy.

As mentioned above, a further object of the invention is a method for predicting cancer treatment responsiveness, wherein said cancer treatment is chosen from immunotherapies, targeted therapies, chemotherapies, and/or radiotherapies and wherein said method comprises:
(a) detecting the expression of type III interferon in a sample from a subject; and
(b) deducing therefrom if the subject will reply positively to a cancer treatment by immunotherapy, targeted therapy, chemotherapy, and/or radiotherapy.

In the scope of the invention, the expression of type III interferon in said sample indicates that the subject will reply positively to a cancer treatment by immunotherapy, targeted therapy, chemotherapy, and/or radiotherapy.

As used herein, the term "subject" refers to a warm-blooded animal such as a mammal, animal or human, in particular a human, who is afflicted with, or has the potential to be afflicted with a cancer as described herein.

As used herein, the term "sample" means a substance of biological origin. Examples of samples according to the invention include, but are not limited to bodily fluids samples and biopsy. Bodily fluids include blood, urine, saliva or any other bodily secretion or derivative thereof. As used herein "blood" includes whole blood, plasma, serum, circulating epithelial cells, constituents, or any derivative of blood.

In particular, the sample is either a biopsy or a sample of blood, plasma or serum. The biological sample according to the invention may be obtained from the subject by any appropriate means of sampling known from the person skilled in the art.

In one embodiment, the sample was previously taken from the subject, i.e., the method according to the present invention does not comprise a step of actively taking a sample from the subject. Consequently, according to this embodiment, the method of the invention is a non-invasive method, i.e., an *in vitro* method.

As used herein, the term "detecting the expression" refers to determinate or quantify the expression or presence of type III interferon. Methods for determining or measuring the expression of a marker are well known in the art. The detection and quantification of a marker can be made by any method known by the man skilled in the art and in the context of the invention, the detection and/or quantification of type III interferon is preferably conducted by measuring the type III interferon protein or mRNA, in particular in a biopsy or in a blood, plasma or serum sample.

mRNA of type III interferon can be detected by any method know by the person skilled in art. Typically, the nucleic acid contained in the biological sample may be extracted according to standard methods, for example using lytic enzymes or chemical solutions or extracted by nucleic-acid-binding resins following the manufacturer's instructions. The extracted mRNA may be subjected to coupled reverse transcription and amplification, such as reverse transcription and amplification by polymerase chain reaction (RT-PCR), using specific oligonucleotide primers that enable amplification of a region in said genes. Quantitative or semi- quantitative RT-PCR can be used. Extracted mRNA may be reverse-transcribed and amplified, after which amplified sequences may be detected by hybridization with a suitable probe or by direct sequencing, or any other appropriate method known in the art. For example, mRNA is detected by hybridation *in situ* on tumor section. Other methods of amplification include ligase chain reaction (LCR), transcription-mediated amplification (TMA), strand displacement amplification (SDA) and nucleic acid sequence based amplification (NASBA). mRNA can also be identified by RNAseq or by single cell RNAseq allowing the identification of the cell expressing the mRNA.

In particular, in the context of the present invention, mRNA of type III interferon is detected by RNAseq, single cell RNAseq or hybridation *in situ.*

Type III interferon protein can be detected by any method know by the person skilled in art. It can be performed by immunoassay or immunoblots or by analytical methods, like for example classical Elisa, electrochemiluminescence immunoassays, digital biomarker detection technology, Simoa, mass spectrometry (MS), capillary electrophoresis-mass spectrometry (CE-MS), liquid chromatography coupled to mass spectrometry (LC-MS, LC-MS/MS), quantitative methods with isotopic labeling (stable isotope labeling by amino acids in cell culture (SILAC), isotope coded affinity tags (ICAT), isobaric tag for relative and absolute quantitation (ITRAQ), label-free methods like selective reaction monitoring (SRM) or multiple reaction monitoring (MRM) assays, or bio-molecular interaction analysis/surface plasmon resonance (BIA/SPR) technologies encompassing methods with calibration and without calibration as calibration free concentration analysis for example.

The term "immunoassay" as used according to the present invention includes competition, direct reaction, or sandwich type assays. Such assays include, but are not limited to, immunofluorescence (IF), immunohistochemistry (IHC) on tumor section, agglutination test, enzyme-labelled and mediated immunoassays, such as ELISA, biotin/avidin type assay, radioimmunoassay, immunoelectrophoresis, and immunoprecipitation.

In particular, in the context of the present invention, type III interferon protein is detected by IHC, IF on tumor FFPE section or Elisa on tumor soluble milieu or plasma.

The term "the expression of" refers to an amount or a concentration of a transcription product, for instance mRNA, or a translation product, for instance a protein. Typically, a level of mRNA expression can be expressed in units such as transcripts per cell or nanograms per microgram of tissue. A level of a protein can be expressed as nanograms per microgram of tissue or nanograms per milliliter of a culture medium, for example. Alternatively, relative units can be employed to describe an expression level.

In one embodiment, the method according to the invention also comprises a step (a') of detection of at least one additional biomarker, in particular Dendritic Cells subtypes markers, such as tumor mutational burden (TMB), neoAg load, CD8 T cells infiltration, PDL1 expression, cDC1 mRNA signature, CLEC9A and XCR1 proteins or mRNA, in a sample of said subject.

In particular, said step (a') is a step of detection of the expression of cDC1 mRNA signature or CLEC9A and/or XCR1 proteins or mRNA in a sample of said subject.

Said expression can be detected by the methods previously mentioned.

In one embodiment of the method according to the invention, said Type III interferon is expressed by cDC1.

This can be determined by any method know by the man skilled in the art, for example by double staining or *in situ* hybridization for IFN-λ (1 to 3) and CLEC9A or XCR1.

As detection of type III interferon is predictive of a positive outcome in the treatment of cancer, it is interesting for the physician to know before the treatment, if the tumor of the subject, and in particular the cDC1s have produced type III interferon. In case such method includes the detection of an additional biomarker, it can also be interesting to know if such biomarker is present before treatment.

As a consequence, in one embodiment of the method according to the invention, the detection step(s) (a) and/or (a') are conducted before the start of the cancer treatment with an immunotherapy, targeted therapy, chemotherapy, and/or radiotherapy.

In the case where the type III interferon would not be detected, its production can be induced by a drug able to induce the production of type III interferon.

In that case, the physician could need to check whether or not the tumors, and in particular the cDC1, have produced type III interferon.

In other cases, the physician can have started the treatment with an immunotherapy, targeted therapy, chemotherapy, and/or radiotherapy and would like to follow the production of type III interferon, in particular by the cDC1.

As a consequence, in one embodiment of the method according to the invention, the detection step (a) is conducted both before and after the start of the cancer treatment.

In that latter case, the sample of the subject is preferably a blood, plasma or serum sample.

As a consequence also, in one embodiment of the method according to the invention, the detection of type III IFN is used to select a drug aiming at inducing type III interferon production.

An object of the invention is thus also, a type III interferon inducer for use in combination with an immunotherapy, targeted therapy, chemotherapy, and/or radiotherapy for the treatment of cancer.

In particular, said type III interferon inducer is used to increase the efficacy of cancer treatment with an immunotherapy, targeted therapy, chemotherapy, and/or radiotherapy for the treatment of cancer.

A type III interferon inducer is a drug able induce the production of type III interferon.

It can for example be a PRR agonist such as TLR ligands, STING ligands, RIG-I ligands, or an oncolytic virus.

"PRR agonist" as used herein refers to a Pattern Recognition Receptors agonist. PRRs play a crucial role in the proper function of the innate immune system. PRRs are germline-encoded host sensors, which detect molecules typical for the pathogens. They are proteins expressed, mainly, by cells of the innate immune system, such as dendritic cells, macrophages, monocytes, neutrophils and epithelial cells TLR ligands.

Any agonist known by the person skilled in the art can be used, in particular TLR ligands, STING ligands and/or RIG-I ligands.

"TLR ligands" as used herein refers to Toll Like Receptors ligands. Toll-like receptors (TLRs) are a class of proteins that play a key role in the innate immune system. They include TLR1, TLR2, TLR3, TLR4, TLR5, TLR6, TLR7, TLR8, TLR9, TLR10, TLR11, TLR12, and TLR13. In particular, in the scope of the invention, TLR ligands are TLR3 ligands.

"STING ligands" as used herein refers to Cyclic dinucleotides (CDNs) such as the cyclic guanosine monophosphate-adenosine monophosphate (cGAMP), and the xanthenone derivative DMXAA that activate STING (Stimulator of IFN genes), leading to a potent type I IFN response. Several small molecule immune modulators specifically designed to target and activate human STING are currently under clinical evaluation in several tumor types.

"RIG-I ligands" as used herein refers to agonist of the retinoic acid-inducible gene-I-like receptors. Several immune modulators specifically designed to target and activate human RIG-I are currently under clinical evaluation in several tumor types.

"Oncolytic virus" as used herein has the normal meaning known in the art i.e. a virus that induced lysis of tumor cells but not of normal cells. In the context of the invention, the following examples are contemplated: Pexa-Vec Oncolytic Virus, TG6002, or viral vaccine such as the rotavirus vaccine (Shekarian T et al., "Repurposing rotavirus vaccines for intratumoral immunotherapy can overcome resistance to immune checkpoint blockade ",Sci Transl Med. 2019 Oct 23;11(515). pii: eaat5025. doi: 10.1126/scitranslmed.aat5025; PMID:31645452).

In one embodiment, the tumors of the subject to be treated express cDC1 markers CLEC9A and/or XCR1, and IFN-III is expressed by cDC1.

In another embodiment, the tumors of the subject to be treated do not express type III interferon before treatment but expressed cDC1 markers.

In a further aspect, the present invention also relates to a kit for performing the methods according to the invention, in particular the method for predicting cancer treatment responsiveness, said kit comprising means for detecting the expression of type III interferon in a sample from a subject.

Said means comprises in particular means to detect type III interferon protein or mRNA. Said means are for example means to carry out double in situ hybridization or immune fluorescence on FFPE tumor biopsy section for IFN-λ1, 2 or 3 and one cDC1 marker Clec9A or XCR1; RNAseq on tumor biopsy followed by cDC1 signature deconvolution and IFNI1-3 gene expression analysis; scRNAseq of immune cells from the biopsy allowing simultaneous identification of the cDC1 and their production of IFN-III at single cell level.

In one embodiment, said kit comprises also a control and/ instructions for use.

Instructions for using the kit according to methods of the invention may comprise instructions for processing the sample obtained from the subject and/or for performing the test, or instructions for interpreting the results. A kit may also contain a notice in the form prescribed by a governmental agency regulating the manufacture, use or sale of pharmaceuticals or biological products.

Depending on the procedure, the kit may further comprise one or more of: extraction buffer and/or reagents, western blotting buffer and/or reagents, and detection means.

Protocols for using these buffers and reagents for performing different steps of the procedure may be included in the kit.

The different reagents included in a kit of the invention may be supplied in a solid (e.g. lyophilized) or liquid form.

The kits of the present invention may optionally comprise different containers (e.g., vial, ampoule, test tube, flask or bottle) for each individual buffer and/or reagent. Each component will generally be suitable as aliquoted in its respective container or provided in a concentrated form. Other containers suitable for conducting certain steps of the disclosed methods may also be provided. The individual containers of the kit are preferably maintained in close confinement for commercial sale.

In the scope of the present invention, it has to be understood that "a type III interferon inducer for use" and "a type III interferon for use" are equivalent to "the use of a type III interferon inducer" and "the use of a type III interferon".

The present invention will be further illustrated by the following figures and examples.

### Figures

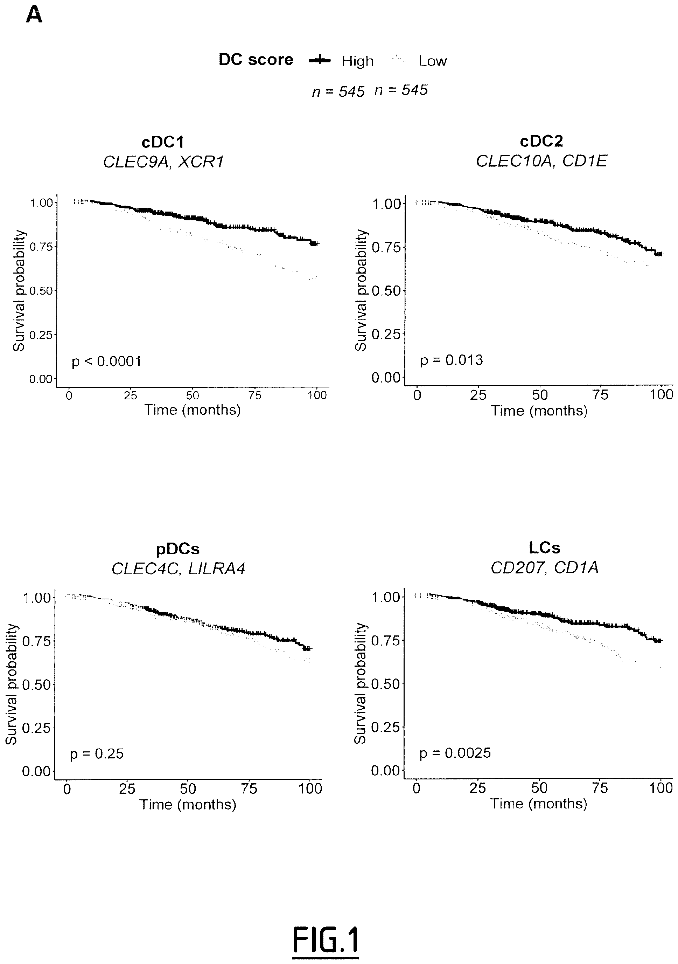

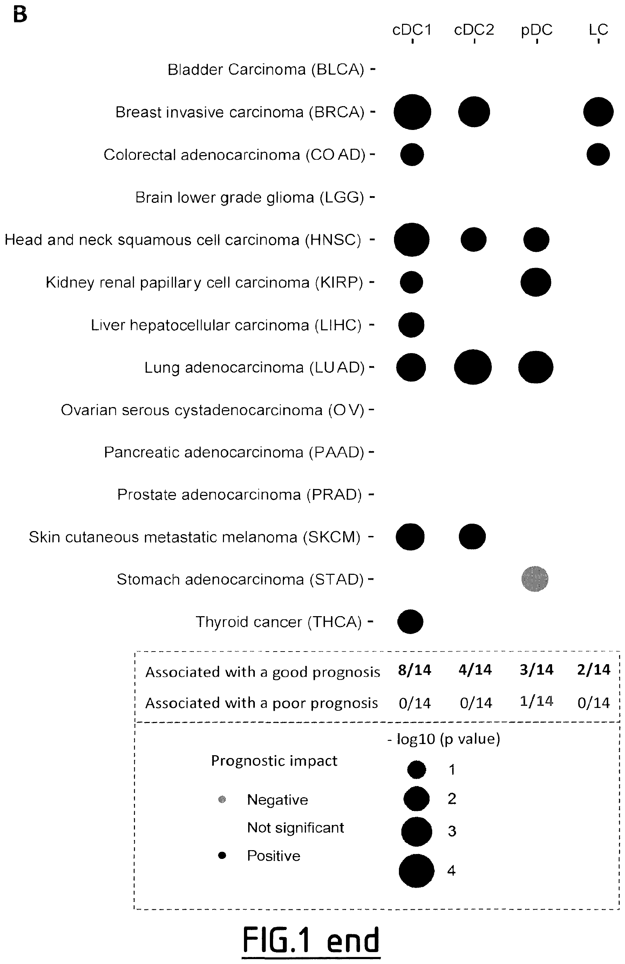

**Figure 1** **:** TA-cDC1 are strongly associated with an increased overall survival in multiple human cancers. **(A)** Kaplan-Meier analysis of the overall survival of patients stratified according to the median for the expression score of each DC subset infiltrating breast tumors (calculated with the MCP-counter algorithm) using TCGA data sets. Statistical analysis: log rank test. **(B)** Summary of p values associated with the log rank test evaluating the prognostic impact of each DC subset in 14 human TCGA cancer data sets.

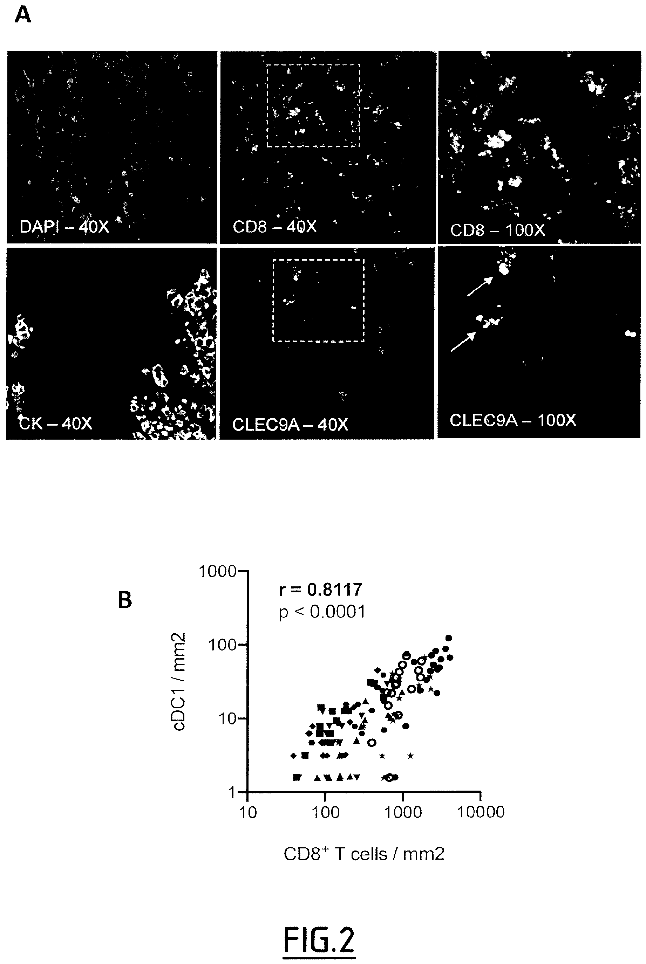

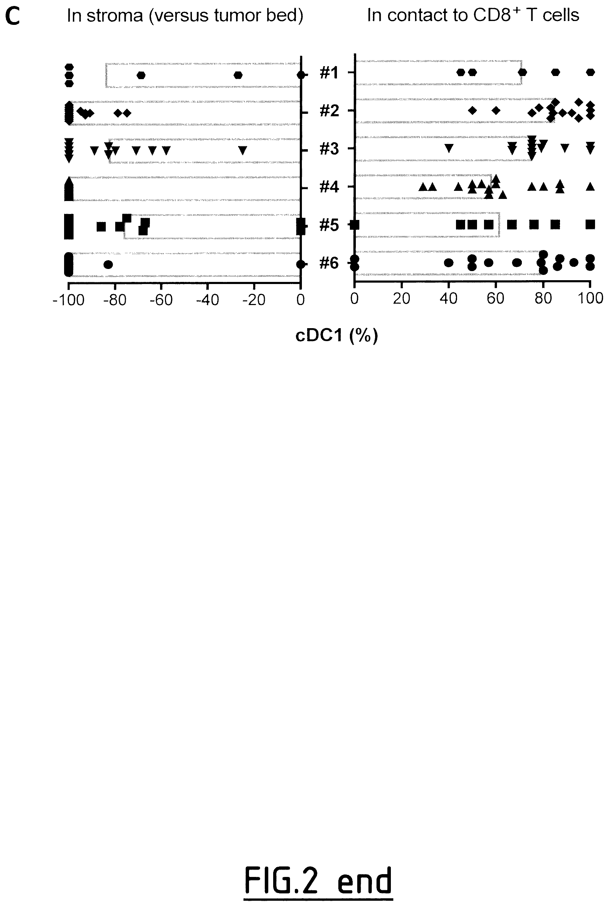

**Figure 2** **:** cDC1 co-localized with CD8 in primary breast tumors. **(A)** Visualization of CD8⁺ T cells and cDC1 respectively by *CD8A* and *CLEC9A* probes *in situ* hybridization, combined to an opal-based immunofluorescent staining of cytokeratin-positive tumor cells. Nuclei were counterstained with DAPI. White scale bars represent 50 µm for 40X images and 25 µm for 100X images. **(B)** Quantification of *CD8*⁺ T cells and *CLEC9A*⁺ cDC1 in 8 breast tumors using the Halo software. Pearson correlation test. **(C)** Proportion of both cDC1 in stroma *versus* tumor bed, and of cDC1 in close contact with at least one CD8⁺ T cell, quantified with the Halo software.

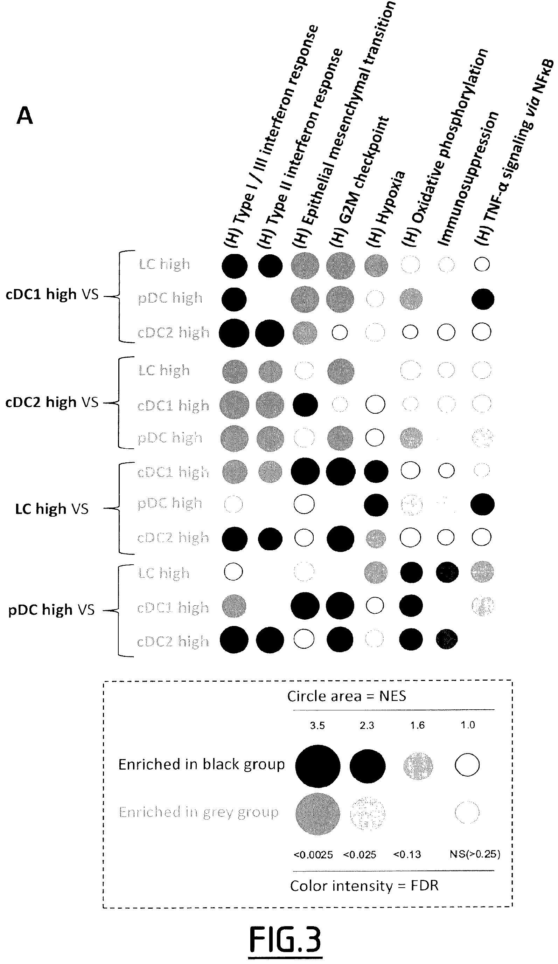

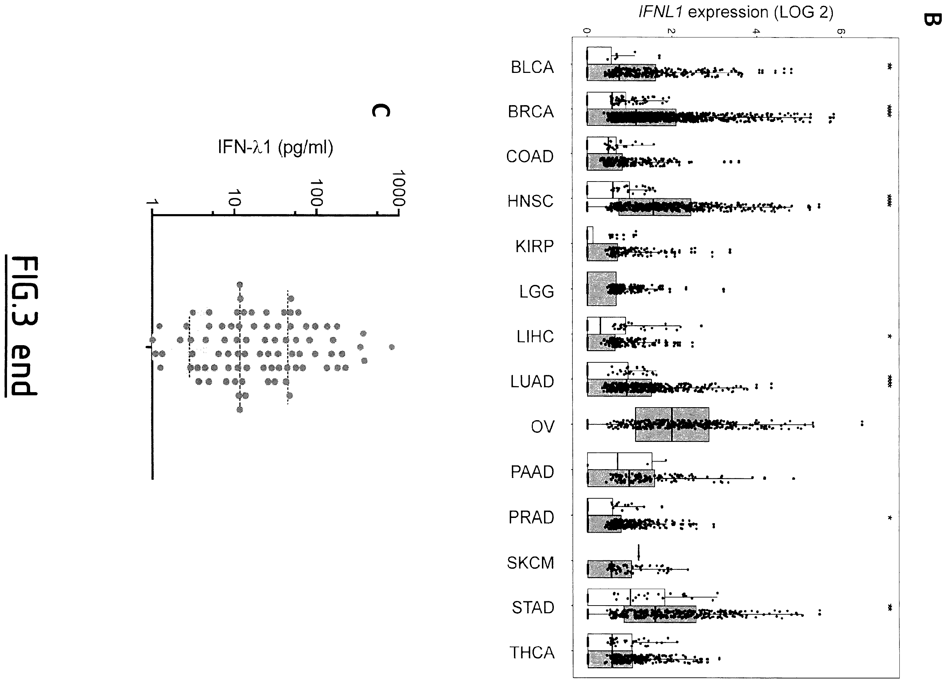

**Figure 3** **:** Enrichment of IFN signatures is a specific feature of cDC1-infiltrated tumors. **(A)** High-throughput GSEA analysis by BubbleGUM in breast tumors enriched in only one DC subset (TCGA data set, n = 48 for cDC1, n = 18 for cDC2, n = 95 for LCs and n = 67 for pDCs). Bubble enrichment patterns (black boxes) highlighted by the selection of gene sets (from Hallmark collection (H) or homemade gene sets) and pairwise comparisons of interest. **(B)** Differential expression analysis of the *IFNL1* gene in multiple transcriptomic TCGA data sets between the tumor (gray boxes) and the normal adjacent tissue (white boxes). Statistical analysis: Wilcoxon test. **(C)** IFN-λ1 quantification by ECLIA multiplex assay in n = 107 STMs of human breast tumors.

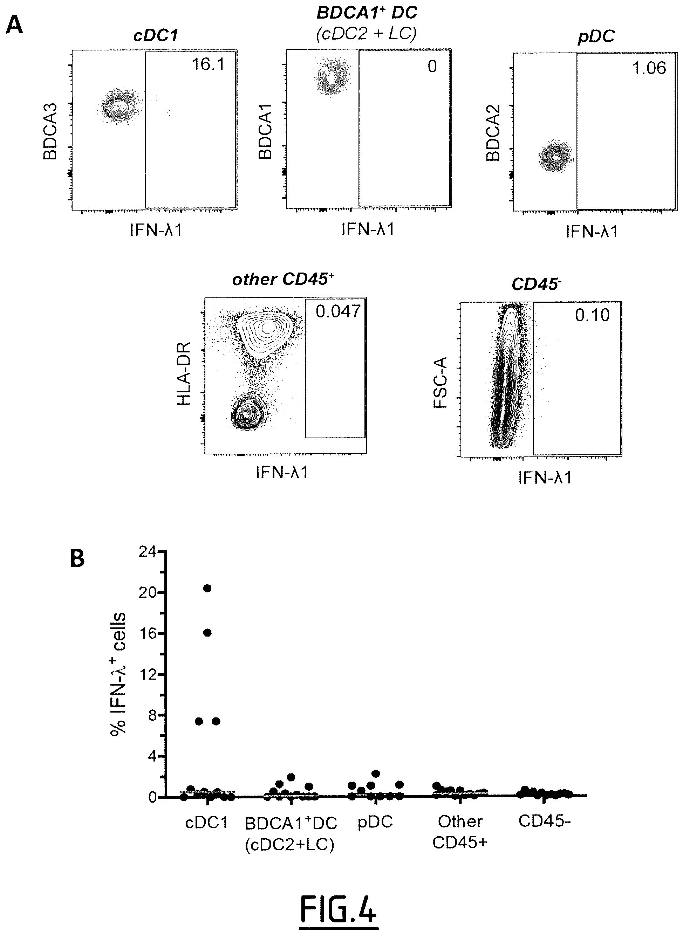

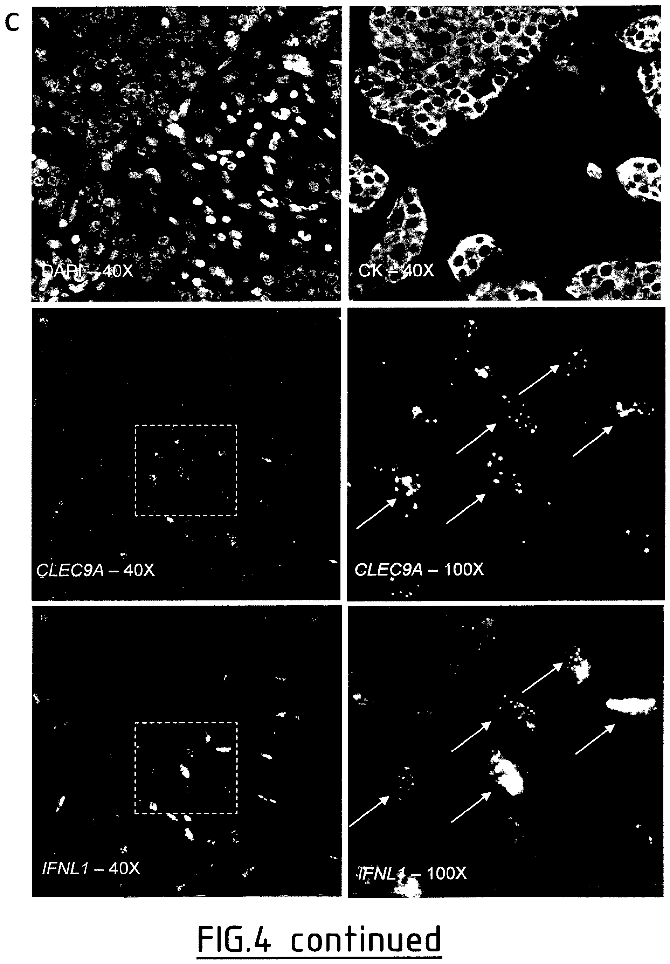

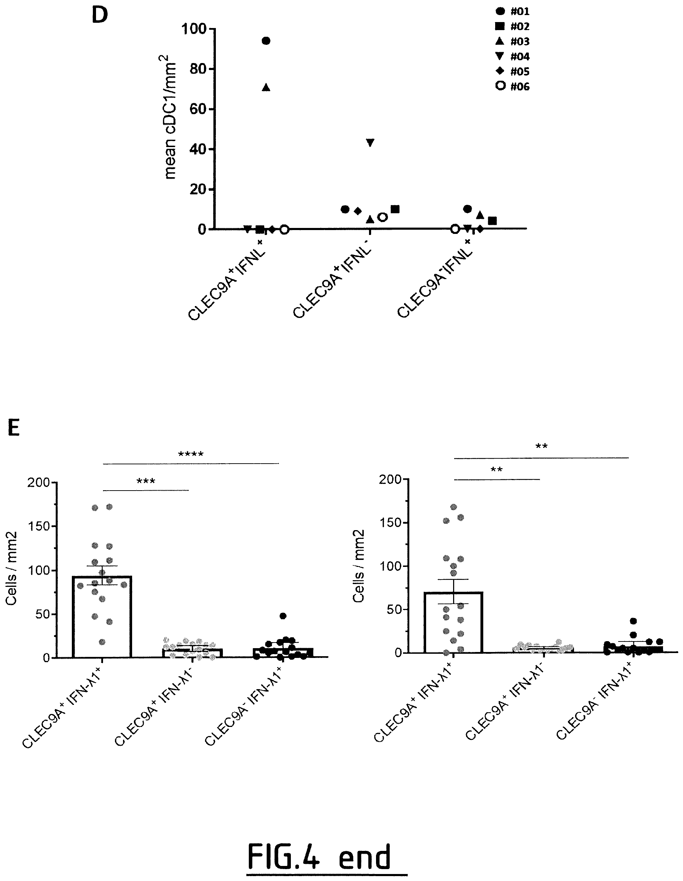

**Figure 4****:** IFN-λ1 is specifically produced by cDC1 in breast tumors. **(A)** Intracellular IFN-λ1 FACS staining of one representative breast tumor suspension positive for IFN-λ1 (grey contour plot = isotype control). **(B)** Proportion of IFN-λ1 producing cells in n = 12 breast tumor suspensions. Horizontal bars represent the median of each group of samples. **(C)** *In situ* detection of IFN-λ1 producing cells: *CLEC9A* (red) and *IFNL1* (green) mRNA were stained by duplex RNAscope, combined to an opal-based immunofluorescent staining of cytokeratin-positive tumor cells (white). Nuclei were counterstained with DAPI. White scale bars represent 50 µm for 40X images and 25 µm for 100X images. **(D-E)** Quantification of *IFNL1* and *CLEC9A* simple and double positive cells in 16 randomly-selected zones of 0.64 mm2 per tumor using the Halo software in 6 breast tumors. The mean for each tumor is represented in **(D)** and the exact quantification of the 2/6 *IFNL1*⁺ tumors (#01 and #03) in **(E).** Bars and error bars respectively represent the mean and the SEM for each group of samples. Statistical analysis: Friedman test. *p<0.05, **p<0.01, ***p<0.001, ***p<0.0001.

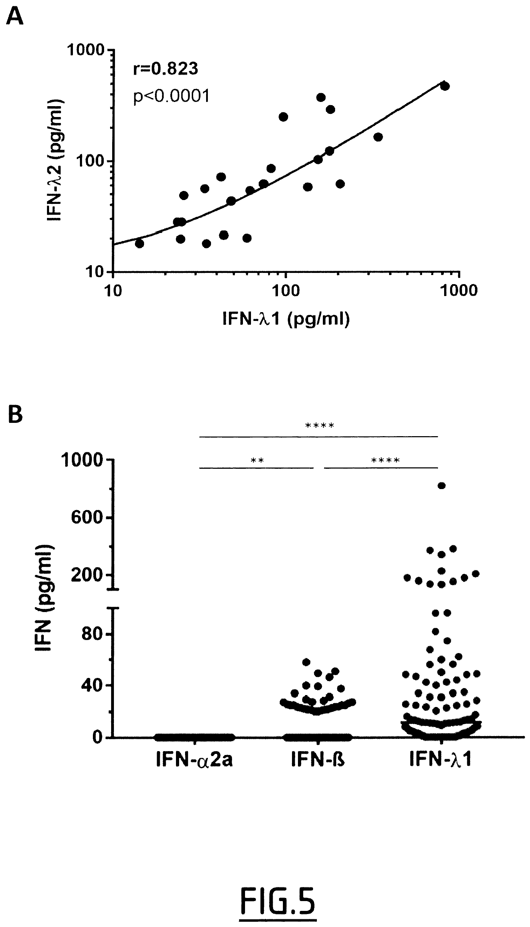

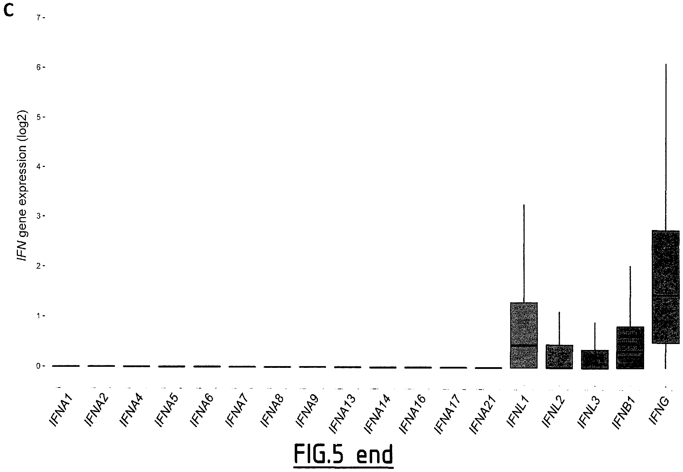

**Figure 5****:** Quantification of IFNs in breast tumors. **(A)** Spearman correlation between IFN-λ1 and IFN-λ2 quantified in breast tumor supernatants by ECLIA multiplex assay. **(B)** IFN quantification by ECLIA multiplex assay in n = 107 STMs of human breast tumors. Statistical analysis by Friedman test. **(C)** IFN gene expression in the TCGA data set of breast cancer (n = 1090 patients).

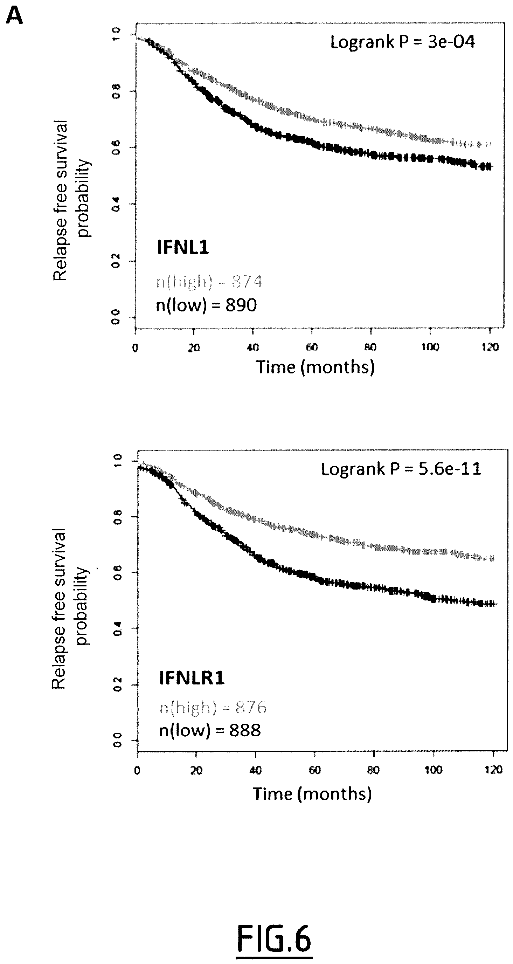

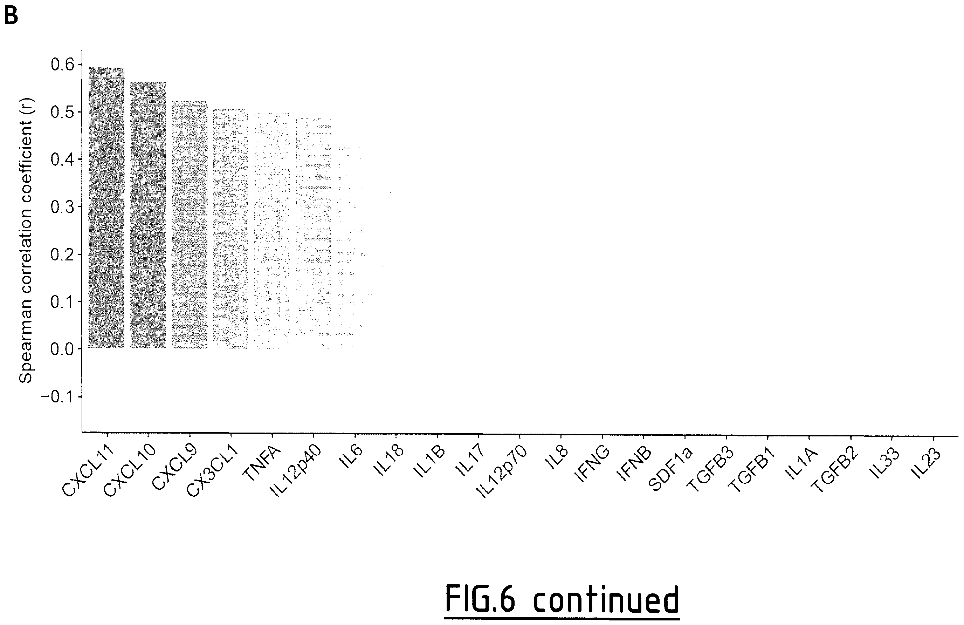

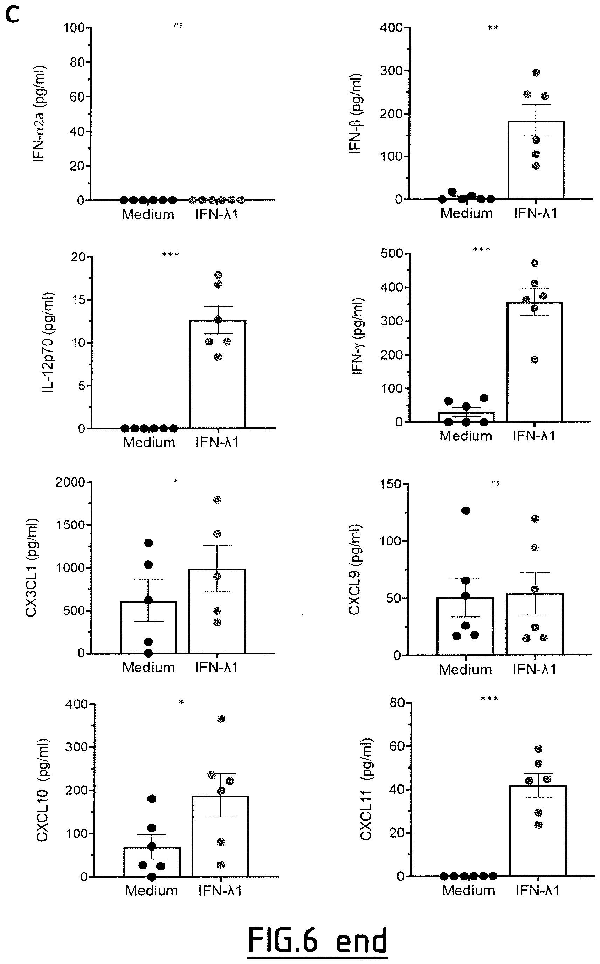

**Figure 6** : IFN-λ1 is associated with a favorable outcome and with a Th1 tumor microenvironment in breast cancer. **(A)** Kaplan-Meier analysis of the relapse-free survival of patients stratified according to the median for the expression of *IFNL1* and *IFNLR1* genes (KMplot transcriptomic data sets). Statistical analysis by log rank test. **(B)** Spearman correlation factors between IFN-λ1 and the other cytokines and chemokines quantified in n = 107 STMs of human breast tumors by ECLIA multiplex assay. **(C)** Cytokine and chemokine quantification by ECLIA multiplex in the supernatants of n = 6 tumor cell suspensions treated or not with IFN-λ1 for 24 h. Bars and error bars respectively represent the mean and the SEM for each group of samples. Statistical analysis: Paired t tests, *p<0.05, **p<0.01, ***p<0.001, ***p<0.0001.

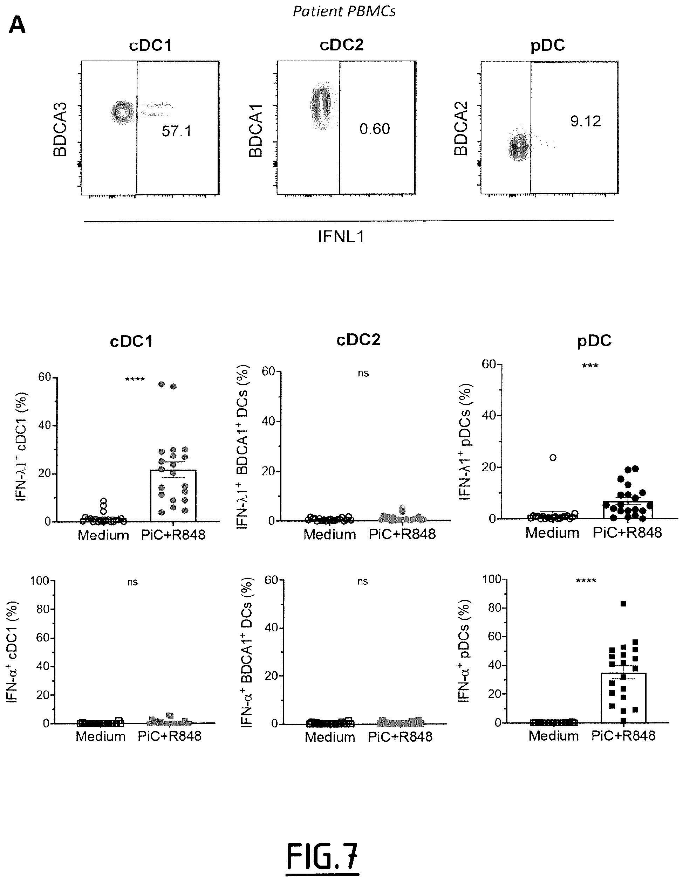

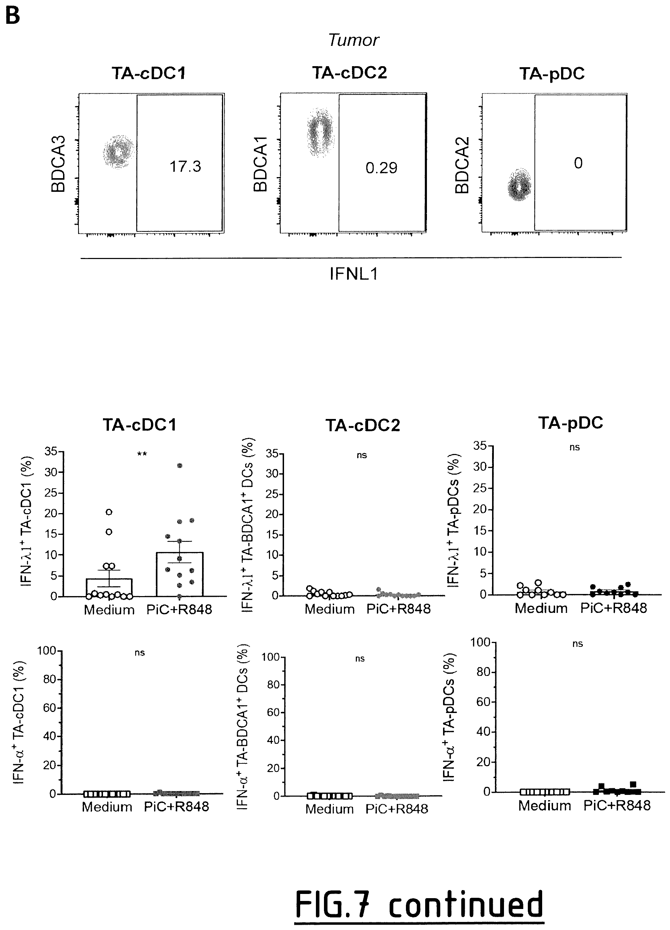

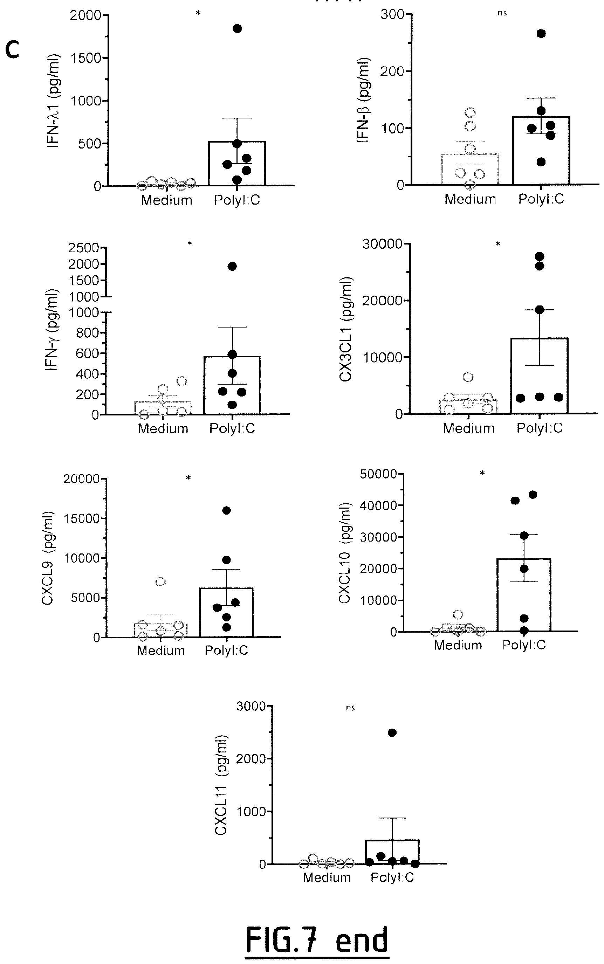

**Figure 7** **:** cDC1 activation by TLR3-L stimulated their IFN-λ1 production and the induction of Th1 immune responses. **(A)** Representative FACS plots of *ex vivo* IFN-λ1 production by DC subsets from patient PBMCs treated or not with TLR-L (Polyl:C + R848) for 5h and proportion of IFN-λ1 and IFN-α producing DC subsets of n = 20 patient PBMCs . Bars and error bars respectively represent the mean and the SEM for each group of samples. Statistical analysis: Paired t test. **(B)** Representative FACS plots of *ex vivo* IFN-λ1 production by DC subsets from breast tumor suspensions treated or not with TLR-L (Polyl:C + R848) for 5h and proportion of IFN-λ1 and IFN-α producing DC subsets of n = 12 fresh breast tumor suspensions. The medium condition corresponds to Fig. 4A. Bars and error bars respectively represent the mean and the SEM for each group of samples. Statistical analysis: Paired t test. **(C)** Multiplex quantification by ECLIA assay of cytokines and chemokines in the supernatants of n = 6 fresh tumor thick sections treated or not with TLR3-L (Poly(I:C)) for 48 h. Bars and error bars respectively represent the mean and the SEM for each group of samples. Statistical analysis: Wilcoxon test, *p<0.05, **p<0.01, ***p<0.001, ***p<0.0001.

### Example

### Materials and Methods

### Breast and ovarian cancer patients

Patients diagnosed with primary breast or ovarian carcinoma were enrolled. Fresh tumors and blood samples (collected in EDTA anticoagulant-containing tubes) were obtained from the Biological Resources Center (BRC) of the Centre Leon Bérard (CLB, BB-0033-00050, Lyon, France) and from the TUMOROTHEQUE (BRC of the Hospices Civils de Lyon). Tumors were used for single cell suspensions preparation or immunohistochemistry analysis on frozen samples. Healthy human blood (collected in EDTA anticoagulant-containing tubes) was purchased anonymously from the Etablissement Français du Sang (EFS, Lyon, France).

### PBMC isolation and tumor cell suspensions

Sections of the resected tumor area selected by the pathologists were placed in RPMI 1640 medium (Gibco) with antibiotics (100 IU/ml penicillin and 100 µg/ml streptomycin, Invitrogen). Tissues were mechanically dilacerated in the same medium. Supernatant of dilacerated tumors were frozen for subsequent cytokine and chemokine quantification. Tissues were then digested for 45 min at 37°C in RPMI 1640 with antibiotics, 1mg/ml of collagenase IA and 20 µg/ml of DNase I (Sigma Aldrich). Digested samples were then filtered on a 70 µm cell strainer and re-suspended in RPMI 1640 with antibiotics and supplemented with 10% FCS (complete RPMI) for further analysis.

Peripheral blood mononuclear cells (PBMCs) were isolated from blood samples of patients or healthy donors through Ficoll density gradient centrifugation (Eurobio).

### Ex vivo stimulation

Tumor cell suspensions and PBMC were cultured at 1x106 cells/ml for 5 h in complete RPMI with different activators: 5 µg/ml R848 (Invivogen) and 30 µg/ml Polyl:C (Invivogen). GolgiPlug (BD Biosciences) was added after 1 h. At the end of the activation, harvested cells were stained, fixed, permeabilized and stained for intracellular cytokines. Tumor cell suspensions were also activated for 48 h with 100 ng/ml IFN-λ1 (R&D) or 100 ng/ml IFN-β (R&D) for cytokine quantification by ECLIA.

To preserve the cyto-architecture, 25 µm sections of fresh tumors were also cut using a vibratome. These sections were incubated for 48 h with 100 µg/ml Polyl:C (Invivogen) and supernatants were collected for cytokine quantification by ECLIA.

### Cell staining and flow cytometry

Single cell suspensions were stained using antibodies listed in Table S1. Dying cells were excluded by Zombie Violet/Yellow staining (Biolegend) depending on the experiment. Lymphocytes, NK cells, neutrophils and other myeloid cells (monocytes, macrophages and inflammatory monocytes) were also excluded using respectively anti-CD3/56/15/14 antibodies in the lineage. Intracellular cytokine or DC-LAMP staining were performed after fixation and permeabilization (Fix/Perm buffers, eBioscience). All flow cytometry acquisitions were done on a LSRFortessa Cell Analyzer (BD Biosciences) and data were processed in FlowJo 10.4 (Tristar). Some flow cytometry data were visualized using viSNE (Cytobank) (65), a dimensionality reduction method which uses the Barnes-Hut acceleration of the t-SNE algorithm. viSNE plots were generated separately for each patient.

### Cytokine and chemokine quantification by MSD assay

The following cytokines and chemokines were quantified in supernatants of dilacerated tumors (SNdil) or supernatants of activated thick tumor sections, using ECLIA (electrochemiluminescence assay) and MSD technology according to the U-plex protocol (MSD): IL-1α, IL-1β, IL-6, IL-8, IL-12/IL-23p40, IL-12p70, IL-17A, IL-18, IL-23p19, IL-33, IFN-α2a, IFN-β, IFN-γ, IFN-λ, CX3CL1, TNF-alpha, CXCL9, CXCL10, CXCL11, TGF-β1, TGF-β2, TGF-β3.

### In situ hybridization (ISH) combined with immunofluorescence on FFPE tumor sections

FFPE tumors were cut into 4µm sections. Then the in situ probe hybridization combined with immunofluorescence was performed on the Leica BOND RX System. This procedure is based on the standard RNAscope LS Multiplex Fluorescent Assay. Tumor slices were rehydrated and deparaffinized before fixation, protease pretreatment, probe hybridization (Hs-CLEC9A-C2 probe in combination with Hs-CD8A-C1 or Hs-IFNL1-C1 probes), amplification and anti-cytokeratin staining. Opal dyes from Perkin Elmer were used for fluorescent detection of probes and anti-cytokeratin antibody. Finally, nucleus were counterstain with DAPI and slides were scanned using the Vectra Polaris automated quantitative pathology imaging system (Perkin Elmer). CLEC9A+ cDC1 and CD8A+ T cells were quantified with the Halo image analysis plateform (Indica labs).

### Survival analysis

The clinical outcome data and RSEM normalized expression datasets from The Cancer Genome Atlas (TCGA) were downloaded from the cBioPortal (provisional data, February 2018) for 14 tumor types: bladder carcinoma (BLCA), breast invasive carcinoma (BRCA), colorectal adenocarcinoma (COAD), brain lower grade glioma (LGG), head and neck squamous cell carcinoma (HNSC), kidney renal papillary cell carcinoma (KIRP), liver hepatocellular carcinoma (LIHC), lung adenocarcinoma (LUAD), ovarian serous cystadenocarcinoma (OV), pancreatic adenocarcinoma (PAAD), prostate adenocarcinoma (PRAD), skin cutaneous metastatic melanoma (SKCM), stomach adenocarcinoma (STAD) and thyroid cancer (THCA). MCPcounter was used to estimate the relative abundance of several population of immune cells. This algorithm originally allows the quantification of the absolute abundance of eight immune populations and two stromal cell populations in heterogeneous tissues from transcriptomic data. Here, in addition to the original cell population signatures defined by Becht et al, the following gene signatures were used to run MCPcounter: cDC1 (CLEC9A, XCR1), cDC2 (CLEC10A, CD1E), pDCs (LILRA4, CLEC4C), LC (CD207, CD1A). Overall survival analyses and plots were performed with R, using the packages survival and survminer. For each immune population, we compared patients displaying the top 50% highest level of infiltration by the given immune cell type and those with the 50 % lowest level. The log-rank test was used to determine statistical significance for overall survival between this two groups of patients. Analysis of progression-free survival was performed for the top 50% and bottom 50% IL29 gene expression ranked values using the Kaplan Meier plotter software.

### Gene expression analysis

RNAseq data for 14 types of solid human cancers and matched normal samples were downloaded using the TCGABiolinks R-package (open access data from the TCGA data portal, https://gdc.cancer.gov, version March 2018) with the harmonized option (data aligned to hg38). For each cancer type, HT-Seq raw read counts were normalized using the DESeq2 R-package and log2 transformed. Wilcoxon tests were performed to assess whether IL29 was differentially expressed between tumor and normal samples.

Heatmaps and hierarchical clusterings. Heatmaps of Log2-normalized expression values of selected genes were performed using the Morpheus website from the Broad Institute (https://software.broadinstitute.org/morpheus/). Hierarchical clusterings were performed using the One-Pearson correlation as a metric and the complete linkage as a clustering method for genes.

### Gene set enrichment analysis

High-throughput gene set enrichment analyses were performed using the BubbleMap module of BubbleGUM (40). BubbleMap analysis was performed with 1,000 gene set-based permutations, and with "Signal2noise" as a metric for ranking the genes. The results are displayed as a bubble map, where each bubble is a GSEA result and summarizes the information from the corresponding enrichment plot. The color of the Bubble corresponds to the tumors from the pairwise comparison in which the gene set is enriched. The bubble area is proportional to the GSEA normalized enrichment score. The intensity of the color corresponds to the statistical significance of the enrichment, derived by computing the multiple testing-adjusted permutation-based P value using the Benjamini-Yekutieli correction. Enrichments with a statistical significance above 0.25 are represented by empty circles. Public gene sets of the Hallmark collection (v6.1) were downloaded from MSigDB and homemade gene sets are detailed in Table S2. Of note, as IFN-III share the same signaling pathway and induces similar ISGs than IFN-I, the type I IFN Hallmark signature was renamed into type I/III IFN signature.

### Statistics

Mann-Whitney tests for paired samples and Wilcoxon tests for unpaired samples were performed for the comparison of two groups. To compare more groups, Kruskall-Wallis tests were performed for unpaired samples and Friedman tests for paired samples. All graphs show each sample and the median value. Statistical significance: *p<0.05, **p<0.01, ***p<0.001, ***p<0.0001

### Study approval

All human samples (blood and tumors) were obtained after approval from the Institutional review board and ethics committee of the CLB (L-06-36 and L-11-26) and patients' written informed consent, in accordance with the Declaration of Helsinki.

### Results

### cDC1 positively correlates with patient's survival in many human cancers

The prognostic impact of cDC1 compared to other DC populations was investigated. Based on available transcriptomic data set of human DCs , human DC signatures composed of *CLEC9A* and *XCR1* for cDC1, *CLEC10A* and *CD1E* for cDC2, *CD1A* and *CD207* for LCs, *CLEC4C* and *LILRA4* for pDCs were derived. The abundance scores for each DC population were estimated in gene expression data sets from The Cancer Genome Atlas (TCGA) with the MCP-counter algorithm developed by Becht *et al.* Then, the overall survival of patients stratified according to the median of each expression score was assessed. This approach revealed the strong association between cDC1 infiltration and a good prognosis in breast cancer (Figure 1A). Interestingly, cDC2 and LCs, but not pDCs, were also positively correlated with patients' survival although to a much lower significance. This analysis was extended to 13 other human cancers and revealed that cDC1 represented the only DC subset associated with a prolonged overall survival in the majority of solid tumors (8/14) (Figure 1B, and data not shown).

### The type I / III interferon response is enriched in tumors with a high cDC1 score

As cDC1 is the key DC population responsible for effector CD8⁺ T cell activation, their precise localization within breast tumors was investigated using *CLEC9A* and *CD8A* probes by *situ* hybridization, combined to an opal-based immunofluorescence detection of cytokeratin-positive tumor cells (Figure 2A). Using the Halo software, 16 zones were randomly defined and quantified for 8 breast tumors. Interestingly, this approach revealed a strong correlation between cDC1 and CD8⁺ T cells (Figure 2B). It was also observed that cDC1 (*CLEC9A*⁺ cells) were predominantly localized in the stroma than in the tumor bed (Figure 2C). Image analysis also highlighted for the first time close contacts between cDC1 and CD8⁺ T cells in breast tumors (Figures 2A, C). Indeed, 70% +/- 10% of cDC1 in stroma are in contact with at least one CD8⁺ T cells (Figures 2A, C). Using TCGA transcriptomic database of breast cancer, a positive correlation was validated between cDC1 and CD8⁺ T cell infiltration scores (data not shown). However, this was clearly not a specific feature of cDC1, since this correlation was shared with cDC2 and pDCs. In contrast, LCs and CD8⁺ T cell infiltration scores were not correlated in tumors, although LCs but not pDCs were associated with a good prognosis (Figure 1A and data not shown).

In order to gain further insight into the functional specificity of TA-cDC1 associated with their positive prognostic impact, the DC infiltration scores defined by MCP-counter was used to design groups of tumors enriched only in one DC subset. Their association with gene signature pathways was analyzed using BubbleGUM for high-throughput gene set enrichment analysis (GSEA) of homemade gene sets and of the MSigDB collections. Interestingly, type I/III and type II IFN signatures were strongly enriched in tumors highly infiltrated with cDC1 compared with other groups (Figure 3A and data not shown). Hierarchical clustering of type I/III IFN signature genes specifically enriched in cDC1^{high} tumors compared with all other tumor types revealed two groups of genes. The first one (group I) is shared with pDCs^{high} tumors and mostly composed of ISGs, and a second one (group II) containing genes only specific of cDC1 enriched-tumors such as *LPAR6, UBA7* or *CSF1* (data not shown). In the type II IFN signature, many genes involved in Ag processing and presentation were highly expressed in cDC1^{high} tumors (*B2M, PSMB8, HLA-A, HLA-B, TAPBP, PSMB9, PSME1*), in addition to genes important for the crosstalk between NK cells and DCs such as *HLA-A* and *-B, IL-15R* and *IL-15.* Interestingly, *CD274*/*PD-L1* was particularly enriched in cDC1^{high} tumors (data not shown). Although no pathway was specifically enriched in cDC2^{high} tumors, these tumors seem to have the lowest IFN signatures (Figure 3A). The signatures related to G2M checkpoint and hypoxia were enriched in LC^{high} tumors compared with other groups (Figure 3A). On the other hand, while the TNF-α signaling pathway was predominantly associated with cDC1, cDC2 or LCs compared with pDCs, oxidative phosphorylation and immunosuppressive pathways were distinctive of pDC^{high} tumors (Figure 3A). This last result is in line with the correlation between pDCs and regulatory T cells (Tregs) in breast tumors compared with other DC subsets (r = 0.6042) (data not shown), and corroborates previous observation. Finally, in comparison with cDC1^{high} tumors, tumors highly infiltrated by pDCs, LCs or cDC2 presented with a more mesenchymal phenotype, as evidenced by the enriched EMT signature (Figure 3A and data not shown). This *in silico* analysis revealed an association between cDC1 and the presence of a strong IFN signature in human breast tumors.

### IFN-λ1 is selectively produced bv cDC1 in breast tumors

IFN-III production is a well-known feature of cDC1 that has been characterized during viral infections, but neither the presence of this cytokine nor its role have been investigated in human tumors. The enrichment in the type I/III IFN signature in cDC1-exclusive tumors prompted the inventors to analyze the link between IFN-III and TA-cDC1. It was initially demonstrated the upregulation of IFNL1 gene expression in tumoral compared with normal adjacent tissue (NAT), based on TCGA transcriptomic data sets of multiple cancers (Figure 3B), confirming the presence of IFN-III in tumors. Interestingly, this differential expression between tumor and NAT was highly significant for breast, head and neck and lung cancers in which cDC1 are strongly associated with favorable patient outcome (Figure 1B). The presence of IFN-λ1 was confirmed at the protein level in 87/106 soluble tumor milieu (STMs) samples obtained by mechanical dissociation of breast tumors, with a concentration ranging from 10 to 800 pg/mL in half of the tumors (Figure 3C). Of note, IFN-λ2 was also detected in these STMs and highly correlated with IFN-λ1 (Figure 5A). In addition, IFN-λ1 was the most abundant IFN subtype in breast tumors compared with type I IFNs (Figure 5B). Indeed, IFN-α was completely absent and IFN-β was detected at a very low concentration (< 50 pg/mL) in only 30% of STMs (Figure 5B), consistent with previous demonstration of the inability of TA-pDCs to produce IFN-α in breast tumors. These results are in complete agreement with mRNA levels of all IFN subtypes in the TCGA breast cancer data set, showing high expression of genes coding for IFN-III, whereas all of the *IFNA* genes were below the detection threshold (Figure 5C). Intracytoplasmic flow cytometry analysis revealed spontaneous production of IFN-λ1 that is restricted to cDC1 in the absence of any *ex vivo* stimulation in a third of the tumors (Figures 4A-B). Of note, TNF-α production was also detected, mostly by TA-cDC2, but no IFN-α (data not shown). To confirm these findings, double fluorescent RNA *in situ* hybridization was performed with *IFNL1* and *CLEC9A* probes (Figure 4C). Using Halo software, 16 zones of 0.64 mm² were randomly defined and quantified for 6 breast tumors (representing a total area per tumor of -12 mm²). This approach confirmed the presence of *IFNL1* transcripts in *CLEC9A*⁺ TA-cDC1 in 2/6 tumors (Figures 4C, D, E). Of note, tumors in which IFN-λ1 was detected appeared to be more infiltrated by cDC1 (data not shown). In addition, *IFNL1* was absent from cytokeratin-positive tumor cells (Figure 4C). These results clearly indicate that IFN-λ1 production is a specific feature of cDC1 in a human tumor context and that this cytokine may play a central role in their antitumor activity.

### IFN-λ1 is associated with a better survival and induces a Th1 soluble microenvironment in human breast tumors

The prognostic impact of *IFNL1* and of *IFNLR1,* the specific chain of its heterodimeric receptor, using public transcriptomic data sets were explored. Notably, a high expression level of these two genes was correlated with an increased relapse-free survival (RFS) (Figure 6A). To understand the underlying mechanisms explaining the beneficial impact of type III IFN in cancer, the soluble tumor microenvironment was further dissected by quantifying multiple cytokines and chemokines in the supernatant of more than 100 dilacerated breast tumors. Interestingly, IFN-λ1 was strongly correlated with CXCR3-L (CXCL11 / CXCL10 / CXCL9) and CX3CL1 chemokines, as well as TNF-α and IL-12p40 (Figure 6B and data not shown). These results raised the hypothesis that the production of IFN-λ1 by cDC1 in tumors could be associated with cytokines and chemokines involved in the recruitment and activation of cytotoxic lymphocytes (NK cells and CD8⁺ T cells). To test this hypothesis, the impact of IFN-λ1 in the tumor microenvironment was assessed by treating human breast tumor suspensions with IFN-λ1 for 24h. It was showed that IFN-λ1 is a potent inducer of IFN-β but do not induce IFN-α2 (Figure 6C and data not shown). In addition, the level of cytotoxic lymphocytes-attracting chemokines was largely increased, as for two CXCR3-L (CXCL10 and CXCL11) and for CX3CL1 (Figure 6C). Of utmost importance, IFN-λ1 activation alone induced IL-12p70 production, a key cytokine for the Th1 lymphocytes differentiation and activation and effector CD8⁺ T cells, as well as high amounts of IFN-γ (Figure 6C). Altogether these results demonstrate a role of type III IFNs in the induction of Th1 immune responses in human breast tumors.

### TLR3 stimulation is a potent strategy to induce the production of IFN-λ1 by cDC1 and Th1 related immune responses in breast tumors

Given the positive prognostic impact of IFN-λ1 in breast cancer and its putative role in the recruitment and activation of cytotoxic immune cells, it was speculated that the induction of IFN-III could be a potential therapeutic strategy in combination with other immunotherapies. Several studies reported the involvement of TLR3 signaling in the activation of IFN-λ production by cDC1. Thus, patient PBMCs or breast tumor cell suspensions were treated with polyinosinic-polycytidylic acid (poly(I:C), double stranded RNA), a TLR3 agonist to target cDC1, in addition to resiquimod (R848, single stranded RNA) to stimulate other DC subsets through TLR7/8. As expected, this combined activation led to TNF-α production by all patient blood DC subsets (data not shown), IFN-α by pDCs, as well as IFN-λ1 both by cDC1 and by pDCs (Figures 7A). In tumor cell suspensions, the impairment of TA-pDC were confirmed to produce IFN-α and demonstrated their inability to produce IFN-λ1 as well (Figures 7B), in contrast to patient blood pDCs. Interestingly, unlike TA-pDCs, TA-cDC1 were responsive to TLR stimulation and could efficiently produce IFN-λ1 in 11 out of 12 tumors (Figures 7B). This activation also led to TNF-α production by all TA-DCs (data not shown). The global impact of such TLR3 stimulation was also evaluated on the tumor microenvironment. Multiple cytokines and chemokines were quantified in the supernatant of *ex vivo* PolyI:C-stimulated fresh breast tumor thick sections instead of tumor cell suspensions in order to conserve the tissue architecture. A strong induction of IFN-λ1 was observed upon TLR3 triggering Figure 7C). The production of IFN-γ, CXCL9, CXCL10 and CX3CL1 was significantly increased (Figure 7C), thus highlighting the potential of cDC1 stimulation in tumor tissue *via* TLR3-L to induce a microenvironment favorable to recruitment and activation of cytotoxic effector cells.

## Claims

1. Type III interferon for use as a biomarker to predict response to a cancer treatment chosen from immunotherapies, targeted therapies, chemotherapies, and/or radiotherapies.

2. Type III interferon for use according to claim 1 in combination with at least one additional biomarker such as: tumor mutational burden (TMB), neoAg load, CD8 T cells infiltration, PDL1 expression, cDC1 mRNA signature, CLEC9A and XCR1 proteins or mRNA.

3. Type III interferon for use according to claim 2, wherein said at least one additional biomarker is cDC1 mRNA signature or CLEC9A and/or XCR1 proteins or mRNA.

4. A method for predicting cancer treatment responsiveness, wherein said cancer treatment is chosen from immunotherapies, targeted therapies, chemotherapies, and/or radiotherapies and wherein said method comprises:
(a) detecting the expression of type III interferon in a sample from a subject; and
(b) deducing therefrom if the subject will reply positively to a cancer treatment by an immunotherapy, targeted therapy, chemotherapy, and/or radiotherapy.

5. Method according to claim 4, wherein the expression of type III interferon in said sample indicates that the subject will reply positively to a cancer treatment by immunotherapy, targeted therapy, chemotherapy, and/or radiotherapy.

6. Method according to claim 4 or 5, further comprising a step (a') of detection of at least one additional biomarker such as tumor mutational burden (TMB), neoAg load, CD8 T cells infiltration, PDL1 expression, cDC1 Mrna signature, CLEC9A and XCR1 proteins or mRNA, in a sample of said subject.

7. Method according to claim 6, comprising a step (a') of detection of the expression of cDC1 mRNA signature or CLEC9A and/or XCR1 proteins or mRNA in a sample of said subject.

8. Type III interferon for use according to claim 3 or method according to claim 7, wherein said Type III interferon is expressed by cDC1.

9. Method according to any of claims 4 to 8, wherein the detection step(s) (a) and/or (a') are conducted before the start of the cancer treatment.

10. Method according to any of claims 4 to 9, wherein the detection step (a) is conducted before and after the start of the cancer treatment.

11. Method according to any of claims 4 to 10, wherein the expression of type III interferon is detected by measuring the type III interferon protein or mRNA expression in a sample of blood, plasma or serum.

12. Method according to any of claims 4 to 11, wherein the detection of type III IFN is used to select a drug aiming at inducing type III IFN production.

13. A type III interferon inducer for use in combination with an immunotherapy, targeted therapy, chemotherapy, and/or radiotherapy for the treatment of cancer.

14. Type III interferon inducer for use according to claim 12 and 13, wherein said inducer is chosen among a PRR agonist such as TLR ligands, STING ligands, RIG-I ligands or an oncolytic virus.

15. Type III interferon inducer for use according to any of claims 12 to 14, wherein the tumors of the subject to be treated express cDC1 markers CLEC9A and/or XCR1 and IFN-III is expressed by cDC1.

16. Type III interferon inducer for use according to any of claims 13 to 14, wherein the tumors of the subject to be treated do not express type III interferon before treatment but expressed cDC1 markers.

17. Type III interferon for use according to any of claims 1 to 3 and 8, method according to any of claims 4 to 12, or type III interferon inducer for use according to any of claims 13 to 16, wherein said cancer is chosen from solid cancers such as breast cancer, ovarian cancer, melanoma, lung cancer, colorectal carcinoma, Head and Neck squamous cell carcinoma, kidney carcinoma, liver hepatocellular carcinoma, thyroid cancer.
